# EUROPEAN PATENT SPECIFICATION

(11) **EP 0 732 895 B1**
(45) Date of publication and mention of the grant of the patent: **17.05.2000**
(21) Application number: 95902863.0
(22) Date of filing: 07.12.1994
(51) Int. Cl.: A61B 18/12

(54) **DEPILATION**
ENTHAARUNG
EPILATION

(30) Priority: 08.12.1993 GB 9325109
(43) Date of publication of application: 25.09.1996
(73) Proprietor: SLS BIOPHILE LIMITED, LLanelli, Dyfed SA14 8LX (GB)
(72) Inventor: CLEMENT, Robert Marc, Pontardawe, West Glamorgan SA8 3HD (GB); KIERNAN, Michael, Seven Sisters, West Glamorgan SA10 9AW (GB)
(74) Representative: Austin, Hedley William
(86) International application number: GB9402682
(87) International publication number: WO9515725

(56) References cited:
- WO-A-92/19165
- US-A- 3 538 919
- US-A- 4 388 924
- US-A- 4 718 416
- US-A- 5 059 192
- US-A- 5 065 515

## Description

The present invention is concerned with an apparatus for use in a method of depilation of mammalian skin. The invention is characterised in detail in the appended claims.

US-A-3538919 and US-A-4617926 are both concerned with depilation. These patents teach the stepwise irradiation of single hairs or hair follicles; the process described in U.S. 3538919 involves inserting a laser probe within a bore of a fibre optic probe. These processes are time consuming, and can lead to unnecessary discomfort to a person being treated.

US-A-5059192 describes a method of depilation using a ruby laser. This depilation method, and the apparatus to carry out the depilation method described, are in particular intended to remove hair containing melanin by radiating the hair follicle by means of a Q-switched ruby laser. The energy applied is preferably 0.4 J/cm² to 10 J/cm², while the pulse duration is stated to be less than one microsecond (that is, within the range of sub-microsecond pulses as stated at the bottom of column 3 of US-A-5059192). The process is also slow and time-consuming; it requires irradiation essentially on a hair-by-hair basis.

Document US-A-4388924 discloses an apparatus for devitalizing the roots of human hairs of a patient using high intensity, short duration pulses of light having wavelengths with respect to which the skin of the patient is non-absorbative and the hair of the patient is relatively absorbative. A narrow, focused beam of the light is aimed at the epidermis of the patient adjacent the hair such that an extension of the beam intersects the hair root. A short pulse passes through the skin and is absorbed in the hair root, destroying its blood supply. The apparatus employs a manually controlled two-axis positioning system supporting the focusing system that is connected to a laser light source by a flexible fiber optic bundle.

We have now developed an apparatus which alleviates the above problems.

According to the present invention there is provided depilation apparatus for use in a method of depilation of mammalian skin, the apparatus comprising:
(a) a laser source capable of emitting radiation having a wavelength in the range of 600 to 1500nm as a series of pulses such that each of the pulses has a pulse duration in the range of 1 µs to 1 ms, said pulse duration being variable so as to permit irradiation of selected intensity;
(b) means for irradiating an area of mammalian skin with the radiation, so as to be capable of destroying biological material present in the area so as to cause depilation of hair present in the area; and
(c) means for effecting movement of the apparatus relative to the skin so as to cause the radiation to irradiate a succession of juxtaposed zones within the abovementioned area such that substantially all the skin within the area is irradiated by the radiation.

It is preferred that the laser source comprises either a ruby laser (wavelength 694.3nm), a neodymium YAG laser (wavelength 1.064µm) or other laser having a wavelength in the abovementioned (visible red to near infra-red) range. The selection of a laser having a wavelength in the range of 600 to 1500nm is advantageous in that radiation of this wavelength is capable of selectively destroying cells or other subdermal biological material responsible for hair growth, whilst not being substantially absorbed by surrounding cells or tissue.

According to the invention, a laser with a variable pulse duration is used which facilitates irradiation of selected intensity, depending on the required application of the laser.

The irradiation zones are juxtaposed so as to substantially cover the treatment area. The means for effecting movement is preferably such that the successive pulses cause irradiation within that area in a boustrophedon manner, so as to ensure substantially complete irradiation of the treatment area.

It is preferred that the irradiation destroys cells present at the root of individual hair follicles; optionally, the irradiation may further destroy cells present in respective bulge regions of follicles.

## Claims

1. Depilation apparatus for use in a method of depilation of mammalian skin, said apparatus comprising:
(a) a laser source capable of emitting radiation having a wavelength in the range of 600 to 1500 nm as a series of pulses such that each of said pulses has a pulse duration in the range of 1 µs to 1 ms, said pulse duration being variable so as to permit irradiation of selected intensity;
(b) means for irradiating an area of mammalian skin with said radiation, so as to be capable of destroying biological material present in said area so as to cause depilation of hair present in said area; and
(c) means for effecting movement of said apparatus relative to said skin so as to cause said radiation to irradiate a succession of juxtaposed zones within said area such that substantially all said skin within said area is irradiated by said radiation.

2. Apparatus according to claim 1, wherein said laser source comprises a ruby laser capable of emitting radiation having a wavelength of 694.3 nm or a neodymium YAG laser capable of emitting radiation having a wavelength of 1064 nm.

3. Apparatus according to claim 1 or 2, wherein said means for effecting movement is such as to cause irradiation within said area in a boustrophedon manner.

## Patentansprüche

1. Enthaarungsgerät zur Anwendung in einem Verfahren zur Enthaarung von Säugetierhaut, wobei das Gerät
(a) eine Laserquelle umfaßt, die zum Aussenden einer Strahlung mit einer Wellenlänge im Bereich von 600 bis 1500 nm in Form einer Reihe von Impulsen befähigt ist, wobei jeder einzelne dieser Impulse eine Impulsdauer im Bereich von 1 µs bis zu 1 ms hat und die Impulsdauer so variierbar ist, daß eine selektive Intensität ermöglicht wird, sowie
(b) Mittel zur Bestrahlung eines Bereiches von Säugetierhaut mit der genannten Bestrahlung aufweist, die so ausgestaltet sind, daß sie zur Zerstörung von in diesem Bereich vorhandenem biologischen Material in der Weise befähigt sind, daß die depilierende Entfernung von in dem genannten Bereich vorhandenem Haar bewirkt wird, und
(c) Mittel zum Ausführen von Bewegungen des genannten Gerätes relativ zur genannten Haut umfaßt, um zu bewirken, daß die genannte Strahlung eine Aufeinanderfolge von aneinandergrenzenden Zonen innerhalb des genannten Bereiches in der Weise bestrahlt, daß im wesentlichen die gesamte genannte Haut innerhalb des genannten Bereiches durch die besagte Strahlung bestrahlt wird.

2. Gerät nach Anspruch 1, bei dem die genannte Laserquelle einen Rubinlaser, der zur Aussendung einer Strahlung mit einer Wellenlänge von 694,3 nm befähigt ist, oder einen Neodym:YAG-Laser umfaßt, der zur Aussendung einer Strahlung mit einer Wellenlänge von 1064 nm befähigt ist.

3. Gerät nach Anspruch 1 oder 2, bei dem die genannten Mittel zum Ausführen von Bewegungen so ausgestaltet sind, daß die Bestrahlung innerhalb des genannten Bereiches in einer abwechselnd links- und rechtsläufigen Weise bewirkt wird.

## Revendications

1. Appareil d'épilation pour l'utilisation dans un procédé d'épilation de la peau d'un mammifère, ledit appareil comprenant :
(a) une source de laser capable d'émettre un rayonnement ayant un longueur d'onde dans la gamme de 600 à 1500 nm, sous forme d'une série d'impulsions, telles que chacune desdites impulsions a une durée d'impulsion dans la gamme de 1 µs à 1 ms, ladite durée d'impulsion étant variable de façon à permettre l'irradiation à une intensité sélectionnée ;
(b) des moyens pour irradier une surface de la peau du mammifère avec ledit rayonnement, de façon à permettre de détruire la matière biologique présente dans ladite surface, de façon à provoquer l'épilation du poil présent dans ladite surface ; et
(c) des moyens pour effectuer un mouvement dudit appareil par rapport à ladite peau, de façon à forcer ledit rayonnement à irradier une succession de surfaces juxtaposées à l'intérieur de ladite surface, de sorte que sensiblement toute ladite peau à l'intérieur de ladite surface soit irradiée par ledit rayonnement.

2. Appareil selon la revendication 1, dans lequel ladite source de laser comprend un laser à rubis capable d'émettre un rayonnement ayant une longueur d'onde de 694,3 nm ou un laser au néodyme-YAG capable d'émettre un rayonnement ayant une longueur d'onde de 1064 nm.

3. Appareil selon la revendication 1 ou 2, dans lequel lesdits moyens pour effectuer un mouvement sont tels qu'ils provoquent l'irradiation à l'intérieur de ladite surface à la manière d'un boustrophédon.
